# EUROPEAN PATENT SPECIFICATION

(11) **EP 4 549 937 B1**
(45) Date of publication and mention of the grant of the patent: **22.07.2026**
(21) Application number: 23831722.6
(22) Date of filing: 12.05.2023
(51) Int. Cl.: G01N 33/50, A01K 67/027

(54) **ZEBRAFISH ANIMAL MODEL FOR INFLAMMATORY DISEASES AND METHOD FOR SCREENING ANTI-INFLAMMATORY AGENTS USING SAME**
ZEBRAFISCHTIERMODELL FÜR ENTZÜNDUNGSKRANKHEITEN UND VERFAHREN ZUM SCREENING VON ENTZÜNDUNGSHEMMERN DAMIT
MODÈLE ANIMAL DE POISSONS-ZÈBRES POUR MALADIES INFLAMMATOIRES ET PROCÉDÉ DE CRIBLAGE D'AGENTS ANTI-INFLAMMATOIRES L'UTILISANT

(30) Priority: 30.06.2022 KR 20220080838
(43) Date of publication of application: 07.05.2025
(73) Proprietor: Raydel Korea Co., Ltd., Seoul 06735 (KR)
(72) Inventor: CHO, Kyung-Hyun, Daegu 42278 (KR)
(74) Representative: EP&C
(86) International application number: PCT/KR2023/006461
(87) International publication number: WO 2024/005358

(56) References cited:
- CN-A- 110 045 073
- CN-A- 113 413 471
- CN-B- 112 245 596
- JP-A- 2006 519 005
- KR-A- 20110 054 952
- KR-A- 20150 040 766
- WANG YI-XIN ET AL: "Studies on mechanism of free N[epsilon]-(carboxymethyl)lysine-induced toxic injury in mice", JOURNAL OF BIOCHEMICAL AND MOLECULAR TOXICOLOGY, vol. 33, no. 7, 1 July 2019 (2019-07-01), US, pages e22322 - n/a, XP093299625, ISSN: 1095-6670, DOI: 10.1002/jbt.22322
- WU YANG ET AL: "The neurotoxicity of N[epsilon]-(carboxymethyl)lysine in food processing by a study based on animal and organotypic cell culture", ECOTOXICOLOGY AND ENVIRONMENTAL SAFETY, ACADEMIC PRESS, SAN DIEGO, CA, US, vol. 190, 18 December 2019 (2019-12-18), XP085985110, ISSN: 0147-6513, [retrieved on 20191218], DOI: 10.1016/J.ECOENV.2019.110077
- CHO KYUNG-HYUN ET AL: "Anti-Inflammatory Activity of CIGB-258 against Acute Toxicity of Carboxymethyllysine in Paralyzed Zebrafish via Enhancement of High-Density Lipoproteins Stability and Functionality", INTERNATIONAL JOURNAL OF MOLECULAR SCIENCES, vol. 23, no. 17, 4 September 2022 (2022-09-04), pages 10130, XP093104743, DOI: 10.3390/ijms231710130
- CHO KYUNG-HYUN ET AL: "CIGB-258 Exerts Potent Anti-Inflammatory Activity against Carboxymethyllysine-Induced Acute Inflammation in Hyperlipidemic Zebrafish via the Protection of Apolipoprotein A-I", CENTER FOR GENETIC ENGINEERING AND BIOTECHNOLOGY, AVE 31, E/158 Y 190, PLAYA, LA HABANA 10600, CUBA, vol. 24, no. 8, 11 April 2023 (2023-04-11), pages 7044, XP093104745, DOI: 10.3390/ijms24087044
- CHO KYUNG-HYUN ET AL: "Beneficial Effect of Cuban Policosanol on Blood Pressure and Serum Lipoproteins Accompanied with Lowered Glycated Hemoglobin and Enhanced High-Density Lipoprotein Functionalities in a Randomized, Placebo-Controlled, and Double-Blinded Trial with Healthy Japanese", INTERNATIONAL JOURNAL OF MOLECULAR SCIENCES, vol. 24, no. 6, 8 March 2023 (2023-03-08), Basel, CH, pages 5185, XP093246926, ISSN: 1422-0067, DOI: 10.3390/ijms24065185
- CHO KYUNG-HYUN ET AL: "Cuban Sugar Cane Wax Alcohol Exhibited Enhanced Antioxidant, Anti-Glycation and Anti-Inflammatory Activity in Reconstituted High-Density Lipoprotein (rHDL) with Improved Structural and Functional Correlations: Comparison of Various Policosanols", vol. 24, no. 4, 1 February 2023 (2023-02-01), Basel, CH, pages 3186, XP093299600, ISSN: 1422-0067, Retrieved from the Internet <URL:https://www.mdpi.com/1422-0067/24/4/3186/pdf> DOI: 10.3390/ijms24043186
- RUFIAN-HENARES J. A., PASTORIZA S.: "Effect of carboxymethyllysine intake on inflammatory bowel disease", PROCEEDINGS OF THE NUTRITION SOCIETY, LONDON, GB, vol. 72, no. OCE1, 1 January 2013 (2013-01-01), GB , pages E24, XP093124791, ISSN: 0029-6651, DOI: 10.1017/S0029665113000268

## Description

### BACKGROUND OF THE INVENTION

### 1. Field of the Invention

The present invention relates to a method for generating zebrafish embryonic and adult models of inflammation using glycotoxic CML, which is very useful for developing drugs to prevent or treat inflammatory diseases, and for screening anti-inflammatory agents using the same.

### 2. Description of the Related Art

The inflammatory response, a type of immune response, is the body's defense mechanism after being exposed to harmful stimuli such as microorganisms or chemical compounds, and refers to the entire process from removing the stimulus to repairing the damaged tissue. It has been reported that when inflammatory responses occur chronically, inflammatory mediators are excessively secreted, promoting the growth of cancer cells or increasing insulin resistance, thereby worsening arteriosclerosis, and participating in various pathological mechanisms. In the immune system, macrophages play an important role in regulating inflammatory responses and immune function and maintaining homeostasis. Macrophages become more active when stimulated by LPS (lipopolysaccharide). LPS is a complex of lipids and polysaccharides covalently linked to external antigens, and is an endotoxin that mainly exists as a component of the outer membrane of Gram-negative bacteria. Macrophages activated by stimulation promote the secretion of proinflammatory cytokines such as tumor necrosis factor-α (TNF-α), interleukin (IL)-1β, and IL-6. It is known that when these inflammatory mediators are formed, they play a major role in mediating inflammation by being involved in the process of arachidonic acid being converted into leukotriene, thromboxane, and prostaglandin through the action of COX (cyclooxygenase) and in the mass production of NO (nitric oxide), thereby causing fatal damage to the host. Among them, NO, a free radical, is a highly reactive substance that is produced from L-arginine by NOS (NO synthase), and NOS is divided into two groups: cNOS (constitutive NOS) and iNOS (inducible NOS). In particular, it has been reported that iNOS is expressed in various cells and produces large amounts of NO when stimulated by external stimuli or pro-inflammatory cytokines. Excessive NO production is known to trigger hyperimmune system abnormalities that cause inflammatory responses.

Cytokines, which are inflammatory mediators, are a class of growth factors. Cytokines are small molecules released by cells into the blood that allow immune cells to migrate to the site of infection, phagocytize damaged cells, and even penetrate blood vessel walls. Cytokines act as endogenous mediators that assist inflammatory signaling. Cytokines themselves are essential to the immune system, and when foreign antigens are introduced, pro-inflammatory cytokines are released from immune cells to kill and neutralize them. Representative pro-inflammatory cytokines involved at this time include tumor necrosis factor (TNF)-α, interleukin (IL)-1, and interleukin-8 (IL-8), which are mainly produced by activated macrophages and induce an inflammatory response to exogenous pathogens.

Pro-inflammatory cytokines (e.g., interleukin-4 and interleukin-10) maintain the function of living organisms by stopping or attenuating the progression of inflammation. The production of pro-inflammatory cytokines and anti-inflammatory cytokines is tightly regulated by complex mechanisms. Imbalanced production of these two types of cytokines causes many diseases, such as arthritis, kidney disease, skeletal abnormalities, asthma, cancer, sepsis, neurodegeneration, neutrophilic alveolitis, hepatitis, ischemia/reperfusion, and inflammatory bowel disease.

Cytokines cause inflammation, which can lead to swelling, fever, and pain in damaged areas. Overproduction of cytokines is lethal to living organisms. When the immune system is over-activated or out of control due to causes such as infection, CART (Chimeric Antigen Receptor T-cell) therapy, certain drugs, or its own immune dysregulation, it releases a large amount of various cytokines, rapidly increasing the levels of a large number of pro-inflammatory cytokines. This phenomenon is called cytokine storm syndrome (CRS). Cytokine storm syndrome is a systemic inflammatory response caused by hyperactivation of the immune system.

The risk of cytokine storm is classified internationally into 5 grades. Grade 1 is a stage where a mild reaction is shown and can be treated with antipyretics, Grade 2 is a stage where a weak reaction is shown within 24 hours, Grade 3 is a stage where a long-term reaction is shown and symptoms can improve but then relapse (renal failure, pulmonary infiltration, etc.), Grade 4 is a stage where life can be at risk, and pressor agents or mechanical ventilation may be required, and Grade 5 is a stage where multiple organ functions deteriorate and are lost in a short period of time and can lead to death due to multiple organ failure.

Known specific symptoms of cytokine storm syndrome include fever, headache, skin rash, joint pain, muscle pain, hypotension, vascular leakage, disseminated intravascular coagulation, and even multiple organ failure. In addition to cytokine levels, associated indicators of cytokine storm syndrome include lymphopenia, increased creatinine, disturbances in coagulation parameters, and increased ferritin and C protein. Clinically, common causes of cytokine storm syndrome include CART therapy, H5N1, H1N1, SARS, MERS, and COVID-19, and the cytokines involved primarily include TNF-α, IL-1, IL-2, IL-6, IL-12, IFN-α, IFN-β, IFN-γ, MCP-1, IL-8, G-CSF, MCP-1, etc. Currently, the specific pathophysiologic mechanism of cytokine storm syndrome is not clearly understood (presumably related to a cytolytic immune response), and there are no specialized therapeutic drugs to treat it. Currently, most of the experience in the clinical treatment of cytokine storm syndrome comes from immunotherapy, specifically from adoptive cell therapy. Drugs that have been tried to treat cytokine storm syndrome include peroxisome proliferator-activated receptor agonists, sphingosine-1-phosphate receptor agonists, cyclooxygenase inhibitor, antioxidants, anti-tumor necrosis factor therapy, intravenous immunoglobulin, and other therapies.

Acute inflammation or hyperinflammation is associated with cytokine cascades, and is known to be closely related to sudden death due to recent COVID-19 infection and worsening symptoms, pain caused by chronic inflammation, and worsening of autoimmune diseases (rheumatoid arthritis, psoriasis, sepsis, etc.). Glycation, a non-specific, non-enzymatic process in which carbohydrates are attached to proteins, produces advanced glycation end products (AGEs), which in turn cause excessive inflammation, trigger a cytokine storm, and cause nerve paralysis, leading to loss of motor skills.

High-density lipoproteins (HDL) in the blood are well known to have antioxidant and anti-inflammatory effects, but it is known that when HDL becomes glycated, deformed and dysfunctional HDL increases, which actually worsens inflammation. Normal HDL has excellent antiviral activity and can kill COVID-19 virus, but abnormal HDL lost its antiviral activity and showed macrophage death, dermal fibroblast death, and embryotoxicity (Cho, K.H.; Kim, J.R.; Lee, I.C.; Kwon, H.J. Native high-density lipoproteins (HDL) with higher paraoxonase exerts a potent antiviral effect against SARS-CoV-2 (COVID-19), while glycated HDL lost the antiviral activity. Antioxidants 2021, 10, 209).

Among advanced glycation end products (AGEs), N-ε-carboxymethyllysine (CML) is known to promote LDL oxidation sensitivity in diabetic patients, thereby promoting and worsening the progression of arteriosclerosis (Bucala R et al. Modification of low density lipoprotein by advanced glycation end products contributes to the dyslipidemia of diabetes and renal insufficiency. Proc Natl AcadSci U S A 1994;91:9441 - 5). However, there have been no reports on whether CML causes acute neuroparalysis and loss of motor skills.

Patients with type 1 or type 2 diabetes have increased levels of CML in their blood, which promotes the expression of toll-like receptor 4 (TLR-4) and ultimately increases high-sensitivity C-reactive protein (hs-CRP), an inflammatory marker (The Journal of Clinical Endocrinology & Metabolism, 93(2), 578-583*) .*

These patients showed increased concentrations of inflammatory cytokines such as interleukin-1β (IL-1β), tumor necrosis factor-α (TNF-α), and interleukin-6 (IL-6) in their serum, which are associated with the excessive inflammatory response caused by CML. This hyperinflammation leads to a cytokine storm, which may be the cause of acute inflammation and death caused by COVID-19 infection.

Well-known ligands of toll-like receptor-4 (TLR-4) include CML, lipopolysaccharide (LPS), and heat shock protein (HSP)-60, which are known to be increased in diabetic patients. It is well known that diabetic patients have increased levels of not only TLR-4 but also interleukin-6 and TNF-α. Therefore, regulating signal transduction of toll-like receptor (TLR) is well known as a good way to suppress the inflammatory cascade. It is well known that the mechanism by which structural analogues of HDL suppress inflammation inhibits lipopolysaccharide (LPS), which stimulates inflammatory signaling of TLR-4. In addition, recombinant HDL has been reported to have anti-inflammatory effects by reducing the expression of TLR-4 and inhibiting inflammatory signaling by TLR-4.

Additionally, cytokines such as interleukin-1β (IL-1β), tumor necrosis factor-α (TNF-α), and interleukin-6 (IL-6) may be related to neuroinflammation. In general, neuroinflammation accompanies the pathogenesis of various neurological and neurodegenerative diseases, such as cerebral ischemia, Alzheimer's disease, Parkinson's disease, Huntington's disease, and amyotrophic lateral sclerosis. Microglia, the unique immune cells in the brain, play a particularly important role in brain inflammation through the production of pro-inflammatory cytokines, nitric oxide (NO), and other neurotoxic factors. Activated microglia can directly damage neurons by stimulating the secretion of cytokines such as tumor necrosis factor-α (TNF-α) and interleukin (IL)-6, which can cause neurotoxicity and motor neuron paralysis, leading to neurodegenerative diseases.

Although mammals have the advantage of genetic and physiological similarities to humans as animal models for new drug discovery and disease research, they are not suitable for large-scale screening due to technical support problems, high costs, and demanding housing requirements within animal facilities. Therefore, there has been a need for alternative laboratory animals with high fertility, low costs, and simple housing requirements. Since the establishment of the animal model in the early 1980s, zebrafish (Danio rerio) has been developed as an important organism for drug screening. Zebrafish has a significant advantage over other vertebrate models because they are uniquely amenable to screening in 96-well or 384-well plate formats. Furthermore, the zebrafish genome is about 80% homologous to the human genome, giving it an advantage over smaller organism models such as Drosophila melanogaster (60%) and Caenorhabditiselegans (36%).

Compared to other animal models, zebrafish are less expensive, produce a large number of fertilized eggs, allowing for highly efficient research, and have transparent embryos with a short developmental period of 48 hours, allowing for real-time observation. In particular, zebrafish have the advantage of being able to observe specific organs in real time in vivo by expressing organ-specific biomarkers such as fluorescent proteins, and of being able to track at the cell level during the embryonic stage. In addition, since zebrafish undergo external fertilization, it is easy to manipulate their genes by injecting genetic materials into the yolk of fertilized eggs.

Taking advantage of these biological characteristics of zebrafish, zebrafish are widely used as an experimental model. Although there is a prior art document (Korean Patent No. 10-1146821) on constructing a zebrafish animal model for inflammatory diseases in which lipopolysaccharide (LPS), oxidized low density lipoprotein (oxLDL), and glycated apoA-I (gA-1) are treated to cause chronic inflammation, the present invention is the first to disclose a zebrafish animal model for inflammatory diseases using CML carboxymethyllysine (CML).

### [PRIOR ART REFERENCE]

### [PATENT REFERENCE]

(Patent Reference 0001) Korean Patent No. 10-1146821
(Patent Reference 0002) KR 2011 0054952 A, which discloses a zebrafish model for inflammatory diseases and a method for screening anti-inflammatory agents using the model.

### SUMMARY OF THE INVENTION

The present invention is defined by the appended claims.

It is an object of the present invention tc provide a useful method for screening a novel anti-inflammatory agent by injecting carboxymethyllysine (CML), an advanced glycation end product, into zebrafish adults and embryos to induce acute death and developmental disorders in embryos and neuroparalysis in adults, and by simultaneously injecting a test substance that suppresses these effects together with CML to compare the degree and speed of inflammation relief and paralysis recovery.

To achieve the above object, the present invention provides a method for screening a novel anti-inflammatory agent as defined in the appended claims, comprising the following steps:
1) a step of inducing inflammation in zebrafish by treating CML;
2) a step of injecting CML together with test substances into the zebrafish; and
3) a step of confirming the effects of test substances by comparing the zebrafish above with those treated with CML alone.

### ADVANTAGEOUS EFFECT

The present invention provides a method as defined in the appended claims, for screening a novel anti-inflammatory agent in a simpler and more economical manner by using zebrafish adults and embryos and the advanced glycation end product CML to induce embryonic death, acute cytokine storm, and neuroparalysis.

### BRIEF DESCRIPTION OF THE DRAWINGS

Figure 1 is a graph showing the survival rate after microinjection of physiological saline (PBS) and CML into zebrafish embryos.
Figure 2 is a diagram showing the developmental stages of zebrafish embryos 24 and 26 hours after microinjection of CML, showing the areas of developmental disorders and malformations in the embryos.
Figure 3 is a graph comparing the survival rates after co-microinjection of CML and Remsima (Infliximab), and CML and Actemra (Tocilizumab) into zebrafish embryos.
Figure 4 is a diagram showing the developmental stages of zebrafish embryos 24 and 26 hours after co-microinjection of CML and Remsima (Infliximab), and CML and Actemra (Tocilizumab) into zebrafish embryos.
Figure 5 is a set of photographs showing that acute paralysis and neurotoxicity were induced by intraperitoneal administration of CML to zebrafish adults, and that after a certain period of time, the paralysis symptoms of some zebrafish recovered and the ability to swim was regained.
Figure 6 is a graph showing the swimming ability of zebrafish that were acutely paralyzed by intraperitoneal injection of CML into zebrafish adults, compared to the swimming ability of zebrafish that were injected with PBS.
Figure 7 is a set of photographs comparing the degree of inflammatory cell infiltration in liver tissue of zebrafish adults treated with PBS alone and CML alone by hematoxylin-eosin staining.
Figure 8 is a graph comparing the degree of inflammatory cell infiltration in liver tissue of zebrafish adults and the viability recovery rate, when treated with PBS alone and CML alone.
Figure 9 is a set of photographs comparing the ability of the test substances to suppress cytokine storm by the test substances by simultaneously treating zebrafish adults with CML and Remsima (Infliximab) and CML and Actemra (Tocilizumab), and confirming the swimming ability of the zebrafish.
Figure 10 is a graph comparing the swimming ability of zebrafish recovered by the test substances by simultaneously treating zebrafish adults with CML and Remsima (Infliximab), and CML and Actemra (Tocilizumab), respectively, and confirming the swimming ability of the zebrafish.
Figure 11 is a graph comparing the viability recovery rate of zebrafish recovered by the test substances by simultaneously treating zebrafish adults with CML and Remsima (Infliximab), and CML and Actemra (Tocilizumab), respectively, and confirming the swimming ability of the zebrafish.
Figure 12 is a set of photographs showing the liver tissues comparing the degree of inflammatory cell infiltration when Remsima (Infliximab) and Actemra (Tocilizumab) were treated in combination with CML.
Figure 13 is a graph comparing the degree of inflammatory cell infiltration when Remsima (Infliximab) and Actemra (Tocilizumab) were treated in combination with CML.
Figure 14 is a graph comparing the levels of serum total cholesterol after injection of CML alone or in combination with CML and test substances.
Figure 15 is a graph comparing the levels of neutral fat after injection of CML alone or in combination with CML and test substances.
Figure 16 is a graph comparing the levels of serum amyloid A (SAA) after injection of CML alone or in combination with CML and test substances.
Figure 17 is a graph comparing the levels of interleukin-6 (IL-6) after injection of CML alone or in combination with CML and test substances.
Figure 18 is a graph comparing the levels of tumor necrosis factor-a (TNF-α) after injection of CML alone or in combination with CML and test substances.

### DESCRIPTION OF THE PREFERRED EMBODIMENTS

Hereinafter, the present invention is described in detail.

It is well understood by those in the art who has the average knowledge on this field that the embodiments of the present invention are given to explain the present invention more precisely.

The present invention provides a method as defined in the appended claims, for screening an anti-inflammatory agent comprising the following steps:
1) a step of inducing inflammation in zebrafish by treating CML;
2) a step of injecting CML together with test substances into the zebrafish; and
3) a step of confirming the effects of test substances by comparing the zebrafish above with those treated with CML alone.

The zebrafish in step 1) above is characterized by the similarity of its organs to those of mammals and most of its organs develop within a few hours after fertilization, so the time of organ formation and the developmental stage are very rapid. Zebrafish develop very quickly. Particularly, the initial cell division of zebrafish occurs at 15-minute intervals, faster than that of E. coli (20 minutes), gastrulation begins at 6 hours of development and is completed at 10 hours, and eye formation begins after 12 hours. After 24 hours of fertilization, the heartbeat and blood cell flow can be observed, and the major organs and systems are fully formed after 5 to 6 days of fertilization.

In a specific embodiment of the present invention, the zebrafish of step 1) is an embryo.

The initial developmental stages of zebrafish are seven stages in the order of zygote period, cleavage period, blastula period, gastrula period, segmentation period, pharyngula period, and hatching period.

In a specific embodiment of the present invention, a zebrafish model for screening an anti-inflammatory agent constructed by treating zebrafish with CML is provided.

The above CML belongs to advanced glycation end products (AGEs).

Advanced glycation end products are formed by the Maillard reaction, which occurs between an amino acid group such as a lysine residue of a protein and a reducing sugar without the action of an enzyme. Non-enzymatic glycation of proteins is a reaction in which the free amino group of a protein, such as lysine or arginine, react with the carbonyl group of a reducing sugar to form a Schiff base, and the compounds formed at this time then undergo a series of complex reactions, such as condensation, rearrangement, oxidation, cleavage, and cyclization, to create a brown compound (melanoidine), which forms an irreversible advanced glycation end product. Advanced glycation end products are irreversible reaction products, so once they are created, they are not broken down even when blood sugar levels return to normal, but rather accumulate in tissues throughout the lifespan of the protein, abnormally altering the structure and function of the tissue. Collagen, which has a relatively long half-life, is easily glycated and forms cross-links with already formed advanced glycation end products, causing abnormal physicochemical changes in the structure of the skin, such as wrinkles on the face, and other protein connective tissues in the body. In addition, advanced glycation end products are recognized by specific receptors for various cell types and cause diabetic complications such as diabetic retinopathy, diabetic neuropathy, diabetic cataract, diabetic nephropathy, and other diseases such as diabetes, chronic kidney disease, heart disease, vascular disease, and aging.

The irreversibly formed advanced glycation end products include Nε-(1-carboxyethyl)lysine (CEL), Nε-(1-carboxymethyl)lysine (CML), methylglyoxal-derived hydroimidazolone No-(5-hydro-5-methyl-4-imidazolone-2-yl)-ornithine (MG-H1), glyoxal-derived hydroimidazolone (G-H1), argipyrimidine, glyoxal-derived lysine dimer, 1,3-di(Nε-lisinoimidazole salt) (GOLD), methylglyoxal-derived lysine dimer, 1,3-di(Nε-lisino)-4-methylimidazole salt (MOLD), etc.

Said CML is a main advanced glycation end product resulting from the oxidative degradation of the Amadori product, and is thermochemically stable and exhibits a relatively simple structure. CML can be generated by three different reaction pathways. The first pathway is the classical Hodge pathway, in which glucose reacts with a lysine residue in a protein to form an Amadori compound, which then undergoes oxidative degradation to form CML. The second pathway is the Wolff pathway, in which glucose undergoes autooxidation to produce a highly reactive intermediate such as glyoxal, which then react with a protein to form CML. The third pathway is the Nimiki pathway, in which a Schiff base formed by glucose reacting with a lysine residue in a protein undergoes oxidative degradation to form CML without undergoing Amadori potential.

In zebrafish embryos, the microinjected CML causes hyperinflammation, leading to acute embryotoxicity and acute cytokine storm.

In a specific embodiment of the present invention, the zebrafish embryo is an embryo aged 15 to 45 minutes (¼ h to ¾ h hpf (hour post fertilization)) after fertilization.

The average development time of the zebrafish developmental stages is 0 - ¾ h for the zygote period, ¾ - 2 ¼ h for the cleavage period, 2 ¼ -5 ¼ h for the blastula period, 5 ¼ h - 10 h for the gastrula period, 10 - 24 h for the segmentation period, 24 - 48 h for the pharyngula period, and 48 h-72 h for the hatching period.

The zebrafish embryo is at the 2-cell stage to 16-cell stage.

The zygote period of zebrafish is the state in which the egg is newly fertilized through the completion of the first zygotic cell cycle, and during the cleavage period, the egg undergoes cleavage through six cycles of the 2-cell stage, 4-cell stage, 8-cell stage, 16-cell stage, 32-cell stage, and 64-cell stage. The blastula period begins when there are 128 cells, and the cells replicate every 15 minutes, reaching about 1000 cells in 3 hours after fertilization, and at 4 hours after fertilization, the embryo forms a sphere and a dome shape is observed inside. During the gastrula period, the neural plate representing the primitive brain is formed, and cells that form specific neurons of the notochord, axial somite-derived muscles, and the hindbrain are present.

The survival rate of the zebrafish embryos induced with inflammation by step 1) above is 14% to 32%.

In a specific embodiment of the present invention, step 3) is a step of comparing the survival rates of zebrafish embryos of the control group not treated with the test substance and the group treated with the test substance.

If the survival rate of zebrafish embryos is increased in the group treated with the test substance compared to the control group not treated with the test substance, the test substance is an anti-inflammatory agent.

In a specific embodiment of the present invention, step 3) confirms whether normal development of the embryo has occurred by checking the developmental stage, the degree of tail elongation, the pigmentation of the eyes, and the presence or absence of the midbrain-hindbrain boundary (MHB) of the brain.

The above zebrafish embryos remained at the 25-somite stage or less after CML injection, the tail was not extended, the pigmentation of the eyes was unclear, and the midbrain-hindbrain boundary (MHB) of the brain was not observed, so it is judged that malformation was induced.

In addition, the zebrafish embryos co-injected with the test substances and CML were in the primordium-3 to primordium-8 stages after injection, exhibited more than 30-somites, had black pigmentation in the eyes, and had a clear midbrain-hindbrain boundary (MHB), indicating normal development.

More preferably, the zebrafish embryos injected with each of the test substances in combination with CML were at the primordium-4 to primordium-6 stages after injection, exhibited more than 32 somites, had black pigmentation in the eyes, and had a clear midbrain-hindbrain boundary (MHB), so it is judged to have shown normal development.

The midbrain-hindbrain boundary (MHB) is highly conserved and folded in the embryonic brain of vertebrates. The midbrain-hindbrain boundary in zebrafish is formed shortly after closure of the neural tube, accompanied by expansion of the ventricles. The midbrain-hindbrain boundary is formed by bending of the basal surface of the neuroepithelium. The zebrafish midbrain-hindbrain boundary is formed in two steps, the first of which is to reduce the midbrain-hindbrain boundary cells to about 75% of the length of the surrounding cells. The second is basal constriction and apical expansion of a small group of cells contributing to the midbrain-hindbrain boundary. Even in the absence of a dilated ventricle, basal constriction still occurs, so that the midbrain-hindbrain boundary is not formed as a passive result of ventricular dilation.

The segmentation period of zebrafish is the stage in which somites, pharyngeal arch primordia, and ganglia develop, the tail appears, and the development begins from the 1-somite stage, and the total number of somites formed is variable, ranging from 30 to 34 somites. The name of the stage of the pharyngula period, which includes the primordium stage, is derived from the formation of the pharyngeal arch that gives rise to the mandibular bone and gills. At this period, the lengthening of the zebrafish embryo slows, the head compresses, fins form, cellular pigmentation occurs, and finally the circulatory system forms and the heart begins to beat. After 24 hours of fertilization, the primordium-5 stage (24 h) progresses, followed by the primordium-15 stage (30 h) and the primordium-25 stage (36 h). The hatching period is the final stage of the embryonic development, during which organ morphogenesis is fairly complete and the cartilage of the head and pectoral fins has developed.

In a specific embodiment of the present invention, the zebrafish of step 1) is an adult.

The zebrafish adults injected with CML exhibit acute cytokine storm, acute paralysis and neurotoxicity.

In a specific embodiment of the present invention, step 3) is a step of comparing the infiltration of inflammatory cells in the group treated with the test substance and the group not treated by staining the cells, and when the infiltration of inflammatory cells in the group treated with the test substance is reduced, the test substance isan anti-inflammatory agent.

Staining of the cells of zebrafish is performed using hematoxylin-eosin staining to observe the infiltration of inflammatory cells, but is not limited thereto.

In a specific embodiment of the present invention, step 3) includes a step of comparing the survival rate of zebrafish adults in the control group not treated with the test substance and the group treated with the test substance.

When the survival rate of zebrafish adults treated with the test substance is increased compared to the control group not treated with the test substance, the test substance isan anti-inflammatory agent.

In a specific embodiment of the present invention, step 3) is a step of comparing the swimming ability of zebrafish adults of the control group not treated with the test substance and the group treated with the test substance, and when the swimming ability of the group treated with the test substance is increased compared to the control group, the test substance isan anti-inflammatory agent.

In a specific embodiment of the present invention, the effect of the test substance in step 3) is determined by checking the levels of total cholesterol, triglyceride, serum amyloid A, IL-6, and TNF-α, and used to select an anti-inflammatory agent, but not always limited thereto.

When the levels of total cholesterol and triglyceride of the group treated with the test substance are lower than those of the control group not treated with the test substance, the test substance is an anti-inflammatory agent.

When the levels of serum amyloid A (SAA), interleukin-6 (IL-6), and tumor necrosis factor-alpha (TNF-α) of the group treated with the test substance are lower than those of the control group not treated with the test substance, the test substance is an anti-inflammatory agent.

Hereinafter, the present invention will be described in detail by the following examples.

However, the following examples are only for illustrating the present invention, and the contents of the present invention are not limited thereto.

### Example 1: Establishment of acute cytokine storm induction system in zebrafish embryos and comparison of cytokine storm inhibitor effects

### <1-1> Construction of zebrafish embryonic animal model microinjected with CML

The present inventors constructed a zebrafish embryonic animal model microinjected with CML to establish an acute cytokine storm induction system. After confirming under a microscope that the freshly spawned zebrafish embryos were 30 minutes post-fertilization (0.5 hour post fertilization (0.5hpf)), the fertilized embryos that were dividing normally were selected. A capillary glass tube (TW100F-4, World Precision Instruments, Sarasota, FL, USA) was inserted into a micro-glass needle maker (PC-10, Narishege, Tokyo, Japan), and the coil was adjusted to be in the center of the capillary glass tube, and then a needle was made at a temperature of 71.3°C. The tip of the needle was sharpened by cutting it diagonally using a sharp forcep, a hole was made, and 5 µL of injection cocktail solution was added to the microglass needle.

Eggs were densely packed and arranged at appropriate intervals on a 1% agarose gel with grooves of appropriate spacing to facilitate rapid microinjection. A microglass needle was attached to a microinjector (PV-830, World Precision Instruments, Sarasota, FL, USA), 5 µL of mineral oil was spread on a graduated hematocytometer, the needle was placed on top of it, and the contents were injected. The size of the bubble formed was measured and calibrated to the desired volume, after which it was injected into zebrafish embryos at the 2-cell and 4-cell stages.

At this time, in order to minimize errors in the microinjection process, microinjection was performed as much as possible into the yolk area. After microinjection, the embryos were separated from the agarose gel and placed in a Petri dish, which was then filled with 20 mL of egg water (0.02 mg/L methylene blue) and stored at 28°C. All injection processes were performed under a stereomicroscope (SMZ-168, Motic, Hong Kong) at 20x magnification.

### <1-2> Comparison of survival rates of zebrafish embryos by CML microinjection

To compare the survival rates of zebrafish embryos, the inventors prepared a non-injected group (No injection), a PBS-injected control group (PBS control), and a CML-injected group. The CML injection group was microinjected with 20 nL of CML solution (25 mg/mL in PBS), i.e., final 500 ng of CML, into zebrafish embryos, and the survival rate, developmental rate, and changes in developmental morphology of each group were observed for 24 hours.

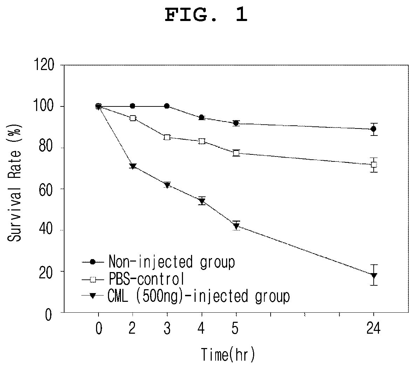

As a result, the non-injected group showed the highest survival rate of 88.9%, and while the PBS-injected control group showed a survival rate of 65.9%, the CML-injected group showed a significantly lower survival rate of 23.5%, indicating acute embryotoxicity caused by CML, an advanced glycation end product (Figure 1). Accordingly, it was confirmed that the CML microinjected into zebrafish embryos caused hyperinflammation, acute embryotoxicity, and acute cytokine storm.

### <1-3> Comparison of embryo development by CML microinjection

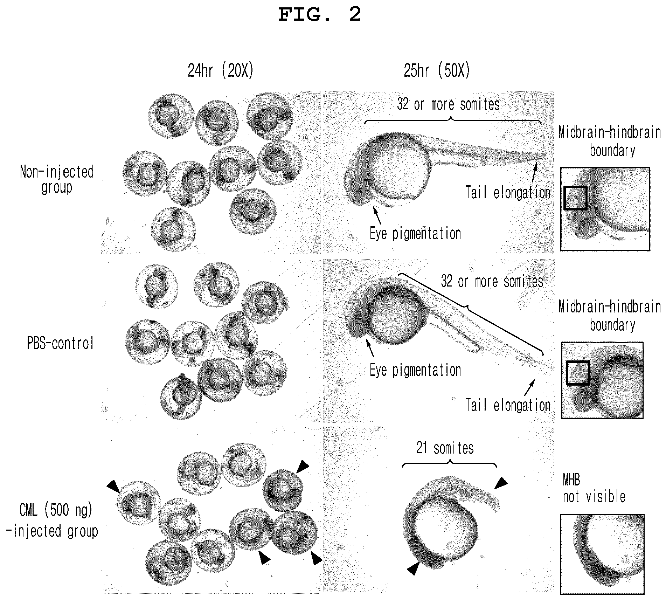

In the case of the PBS control group, 25 hours after PBS injection, the pharyngula period primordium-6 stage, which is the normal developmental rate and morphology seen in the non-injected control group, was shown. However, the CML-injected group exhibited significantly slower development rate, with individuals exhibiting malformed development at 24 hours after CML injection and remaining at the 21-somite stage at 25 hours after CML injection (Figure 2).

The PBS control group showed the same morphology as the primordium-6 at approximately 25 hours post-fertilization as seen in the non-injected control group (No injection). In the PBS control group and the non-injected control group, approximately 34 or more somites were observed, and the eyes were darkened to black due to eye pigmentation (red arrows). In addition, in the PBS control group, the midbrain-hindbrain boundary (MHB) was clearly visible (red solid line). On the other hand, the experimental group injected with CML showed a developmental state of about 19.5 hours after fertilization (21-somite), indicating that they had about 21 somites. In particular, development was slow, developmental malformations were present in the head and tail (blue arrow head), eye pigmentation was very unclear, the tail was not extended, and MHB of the brain was not observed.

When the developmental stage (21-somite), tail extension, and presence or absence of brain MHB of zebrafish embryos were compared with the non-injected group and the PBS-injected control group, it was confirmed that in the case of the CML-injected group, the normal development of the embryos was not achieved and malformations were induced by microinjection of CML into zebrafish embryos.

### <1-4> Comparison of embryonic survival rates by combined microinjection of CML with Remsima (Infliximab) and Actemra (Tocilizumab)

The present inventors used two biopharmaceuticals, Remsima (Infliximab) and Actemra (Tocilizumab), to select a novel drug for suppressing acute cytokine storm caused by CML and recovering neuroparalysis.

Zebrafish embryos were microinjected with 20 nL of CML solution (25 mg/mL in PBS), i.e., final 500 ng of CML, and co-injected with 43 ng of Remsima (Infliximab), a TNF-α inhibitor, and 44 ng of Actemra (Tocilizumab), an IL-6 inhibitor , respectively, and the survival rate, developmental rate, and changes in developmental morphology were observed for 24 hours.

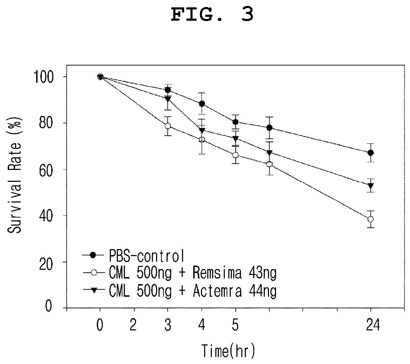

After 24 hours, the survival rate of embryos injected with CML and Remsima (Infliximab) concurrently was 38.4%, and the survival rate of embryos injected with CML and Actemra (Tocilizumab) concurrently was 53.1%. Compared to the PBS injected control group, which showed a survival rate of 67.2%, the survival rate of embryos co-injected with CML and Actemra (Tocilizumab) was confirmed to be higher than the survival rate of embryos co-injected with CML and Remsima (Infliximab) (Figure 3).

### <1-5> Comparison of embryo development by combined microinjection of CML with Remsima (Infliximab) and Actemra (Tocilizumab)

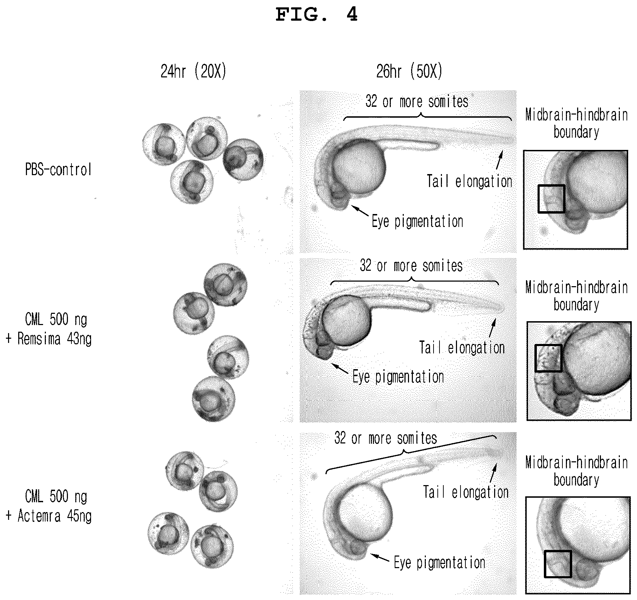

The PBS control group showed normal developmental rate and morphology, and at 25 hours post-injection, exhibited the midbrain-hindbrain boundary (MHB), eye pigmentation, somite number, and tail elongation that are typical of normal development stage at approximately 25-28 hours post-fertilization. Embryos co-injected with CML and Remsima (Infliximab) and embryos co-injected with CML and Actemra (Tocilizumab) showed almost the same rate of development as embryos injected with PBS and no developmental defects (Figure 4).

In the PBS control group, features that appeared at the developmental stage (primo-6) approximately 25 hours after fertilization were observed. In the PBS control group, more than 34 somites were observed, and the eye pigmentation (red arrow), which is the darkening of the eye to black due to pigmentation in the optic cells, and the midbrain-hindbrain boundary (MHB) were clearly visible, and the tail was elongated normally. In the experimental group microinjected with a mixture of CML and Remsima, or CML and Actemra, approximately 32 or more somites were shown, showing developmental rate and characteristics almost same to those of the PBS control group, and no developmental defects were observed (Figure 4).

When CML and Remsima (Infliximab), or CML and Actemra (Tocilizumab) were simultaneously microinjected into zebrafish embryos, the malformed development observed when CML alone was injected was not observed. Thus, the biopharmaceuticals used as the test substances were confirmed to alleviate the inflammatory response and cytokine storm caused by CML, increasing survival rate and inducing normal development.

### Example 2: Establishment of acute cytokine storm induction system in zebrafish adults and comparison of cytokine storm inhibitor effects after paralysis induction

### <2-1> Construction of adult zebrafish animal model microinjected with CML

The present inventors constructed an adult zebrafish animal model injected with CML to establish an acute cytokine storm induction system.

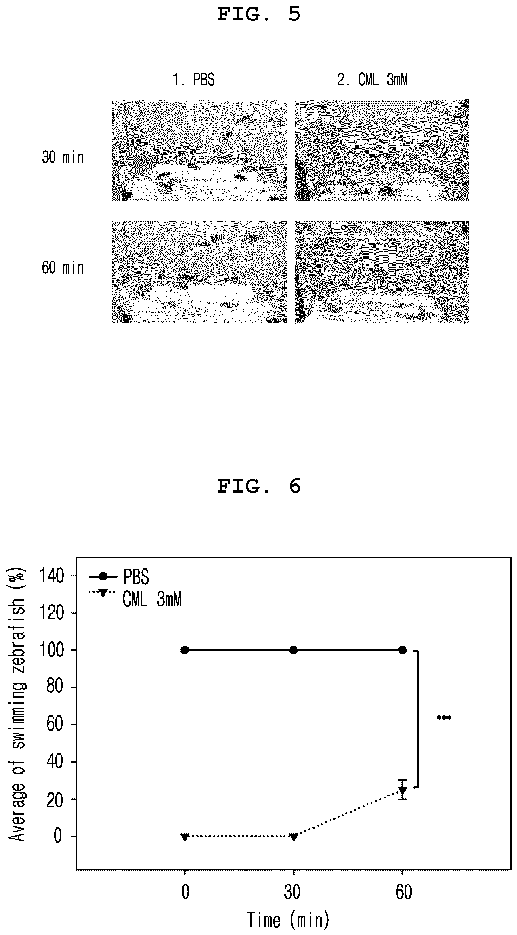

Movements of 10 zebrafish adults (16±3 weeks old) injected intraperitoneally with CML (250 µg, 10 µL) solution (final conc. of CML: 3 mM) and 10 zebrafish adults injected with PBS were observed 30 minutes and 1 hour after injection. Zebrafish of the PBS-injected group were seen swimming actively up and down the tank 30 minutes and 1 hour after injection, and all zebrafish were confirmed to be swimming (Figures 5 and 6). On the other hand, all zebrafish of the CML-injected group were immobilized and lying on the bottom for at least the first 30 minutes after injection. After 1 hour of CML injection, only 25% of zebrafish recovered the ability to swim, but they were less active than zebrafish injected with PBS, and the remaining zebrafish showed acute paralysis and neurotoxicity, losing the ability to swim at 30 minutes and 1 hour after injection (Figures 5 and 6).

Therefore, it was confirmed that injection of CML into zebrafish adults induced acute cytokine storm and acute paralysis and exhibited neurotoxicity.

### <2-2> Comparison of acute cytokine storm induction and therapeutic effects in liver tissue

To compare the degree of acute cytokine storm induction by CML and alleviation, hematoxylin-eosin (H&E) staining was performed on zebrafish adults of the PBS-injected and CML-injected groups, and the degree of inflammatory cell infiltration in the liver tissue was investigated.

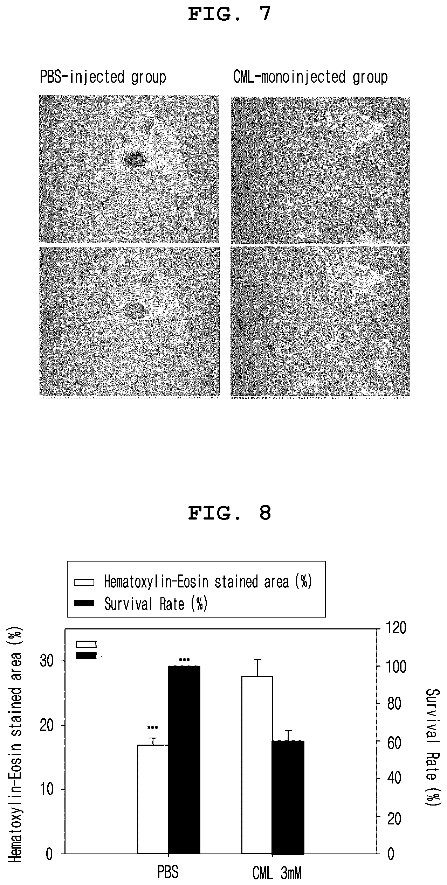

Hematoxylin-eosin staining results showed that the liver tissue of the CML-injected group was darker purple and red and had more inflammatory cell infiltration compared to the liver tissue of the PBS-injected group (Figure 7). In the PBS only injection group, cell infiltration of less than 16% was observed, but in the CML only injection group, inflammatory cell infiltration of 27% was observed, indicating that inflammatory cell activation was increased (Figure 8).

When PBS was injected intraperitoneally into zebrafish adults, a 100% survival rate was observed even after 60 minutes, but the survival rate after 60 minutes of injection of CML alone was less than 49%, demonstrating the extent of death caused by acute cytokine storm (Figure 8).

Therefore, it was confirmed that injection of CML into zebrafish adults induced acute inflammation and acute cytokine storm, and lowered the survival rate.

### <2-3> Comparison of acute cytokine storm induction and therapeutic effects in liver tissue

The present inventors used two biopharmaceuticals, Remsima (Infliximab) and Actemra (Tocilizumab), which are used as treatment agents for rheumatoid arthritis, as test substances to compare the effects of suppressing acute cytokine storm caused by CML and treating neurotoxicity.

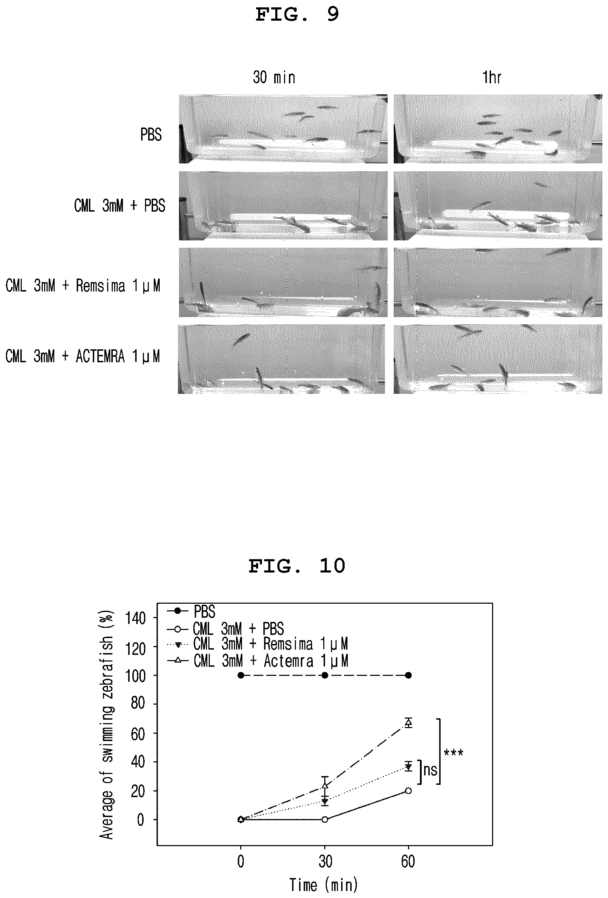

The cytokine storm suppression ability of the test substances was compared by confirming the swimming ability of zebrafish of the groups injected with PBS, CML, CML and Remsima (Infliximab), and CML and Actemra (Tocilizumab) via intraperitoneal administration (Figure 9). All zebrafish of the PBS-injected group were confirmed to be swimming at the top of the tank 30 minutes and 1 hour after injection. On the other hand, zebrafish of the CML group injected intraperitoneally with 3 mM of CML could be seen lying on the bottom of the tank without being able to swim 30 minutes after injection, and 2 to 3 zebrafish showed recovery of swimming ability 1 hour after injection. More zebrafish of the CML and Remsima (Infliximab) injected group and the CML and Actemra (Tocilizumab) injected group were swimming than the CML injected group 30 minutes after injection, and more zebrafish had regained the ability to swim 1 hour after injection, confirming that the test substances suppressed inflammation and alleviated acute paralysis and neurotoxicity.

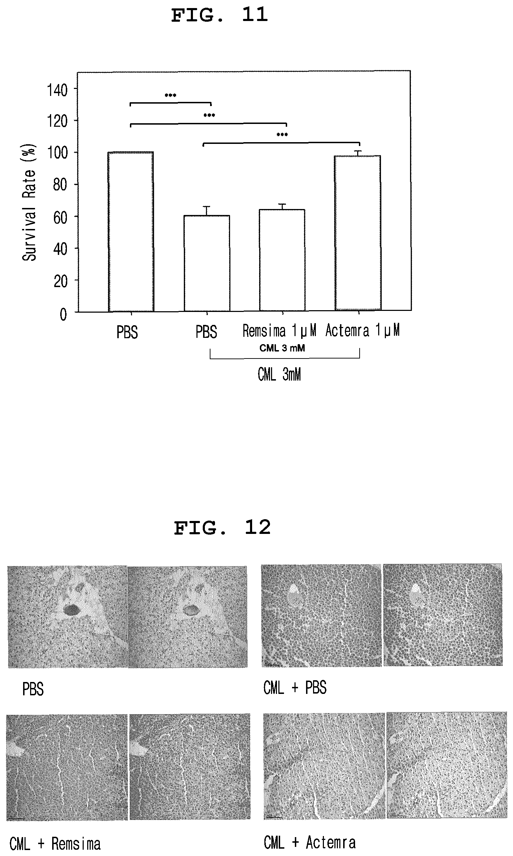

The effects of the test substances in the CML and Remsima (Infliximab) injected group and the CML and Actemra (Tocilizumab) injected group were compared. As a result, when 44 µg of Actemra (Tocilizumab) was injected, recovery of swimming ability was better and survival rate was increased than when 43 µg of Remsima (Infliximab) was injected, showing improvement in acute neurotoxicity in zebrafish that were unable to move, lay on the bottom, and lost swimming ability due to intraperitoneal administration of CML (Figures 10 and 11) .

Zebrafish of the combined treatment group with Actemra (Tocilizumab), an interleukin-6 (IL-6) inhibitor, showed a high survival rate (approximately 97%) and an improvement in cytokine storm, but zebrafish of the combined treatment group with Remsima (Infliximab), a tumor necrosis factor-α (TNF-α) inhibitor, showed a survival rate of 63%, which was similar to the survival rate of 60% in the PBS treatment group, indicating that the effect of suppressing acute cytokine storm and glycation toxicity caused by CML was minimal (Figure 11). Accordingly, it was confirmed that the combined treatment of Actemra (Tocilizumab) was more effective than the combined treatment of Remsima (Infliximab) in alleviating acute paralysis and neurotoxicity and restoring the ability to swim.

### <2-4> Comparison of improving effects of rheumatoid arthritis drugs on acute inflammation in liver tissue

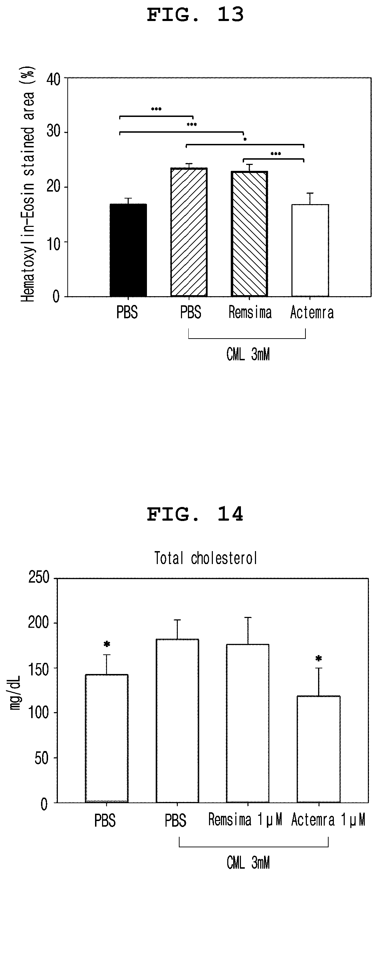

When comparing the infiltration of inflammatory cells using hematoxylin-eosin (H&E) staining, the group treated with CML alone and the group treated with Remsima simultaneously showed a similarly high degree of inflammatory cell infiltration, approximately 23% (Figures 12 and 13). However, in the group treated with Actemra (Tocilizumab) simultaneously, the degree of inflammatory cell infiltration was reduced compared to the group treated with CML alone, showing a significant anti-inflammatory effect, and showed an inflammatory cell infiltration of approximately 15-17%, which is similar to the group treated with PBS alone. That is, the inhibitory effect of the IL-6 inhibitor Actemra (Tocilizumab) on inflammation caused by CML-induced glycation toxicity was greater than that of the TNF-α inhibitor Remsima (Infliximab).

### <2-5> Comparison of ameliorative effects of test substances on increase in total cholesterol and triglyceride caused by CML

Zebrafish adults were treated with CML at a concentration of 3 mM, and Actemra (Tocilizumab) and Remsima (Infliximab) were simultaneously treated to confirm the effects of the test substances on reducing total cholesterol (TC) and triglyceride (TG).

Blood was collected from zebrafish in each group, plasma was isolated, and total cholesterol (TC) and triglyceride (TG) were analyzed. As a result, the group treated with CML alone showed the highest TC and TG concentrations (Figures 14 and 15). These results are consistent with previous findings that increased inflammatory cells in the liver tissue increase total cholesterol and triglyceride in the blood (Feingold, K. R., & Grunfeld, C. (2022). The Effect of Inflammation and Infection on Lipids and Lipoproteins. In K. R. Feingold (Eds.) et. al., Endotext. MDText.com, Inc).

The group treated with PBS alone and the group treated simultaneously with Actemra showed the lowest triglyceride concentration, indicating that the improvement in cytokine storm was most pronounced, which corresponds well with the reduction in inflammatory cell infiltration in the liver tissue. However, the group treated simultaneously with Remsima (Infliximab) showed similar total cholesterol and triglyceride concentrations to the group treated with CML alone, indicating that there was no improvement in lipid profile. This is interpreted to be related to the minimal effect on improving cytokine storm.

### <2-6> Comparison of ameliorative effects of test substances on increase in serum amyloid A, interleukin-6, and tumor necrosis factor caused by CML

Blood was collected from zebrafish in each group, plasma was isolated, and total cholesterol (TC) and triglyceride (TG) were analyzed. Blood was collected from zebrafish in each group, plasma was isolated, and serum amyloid A (SAA), a biomarker of hepatitis induction, interleukin-6 (IL-6), a factor that induces an acute cytokine storm in the liver, and tumor necrosis factor-a (TNF-α), an inflammatory cytokine involved in liver inflammation were analyzed.

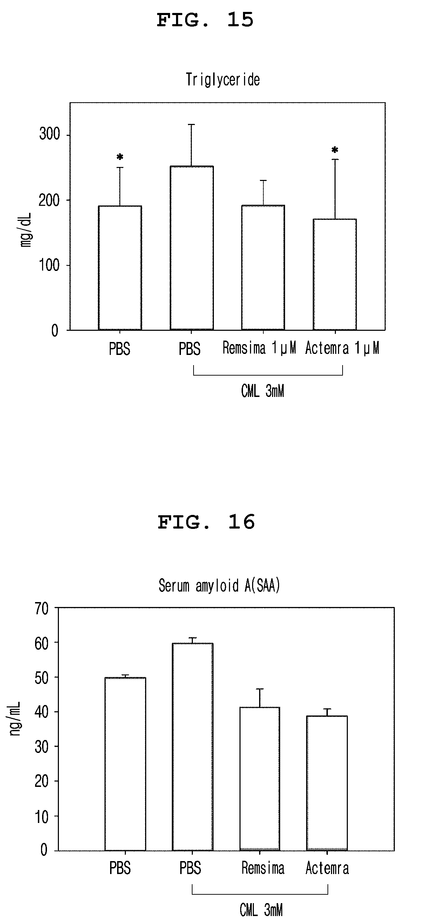

As a result, serum amyloid A (SAA) was highest in the group treated with CML alone at 59.7±1.6 ng/mL, second highest in the group treated with Remsima (Infliximab) at 41.2±5.3 ng/mL, and lowest in the group treated with Actemra (Tocilizumab) at 38.8±2.0 ng/mL (Figure 16).

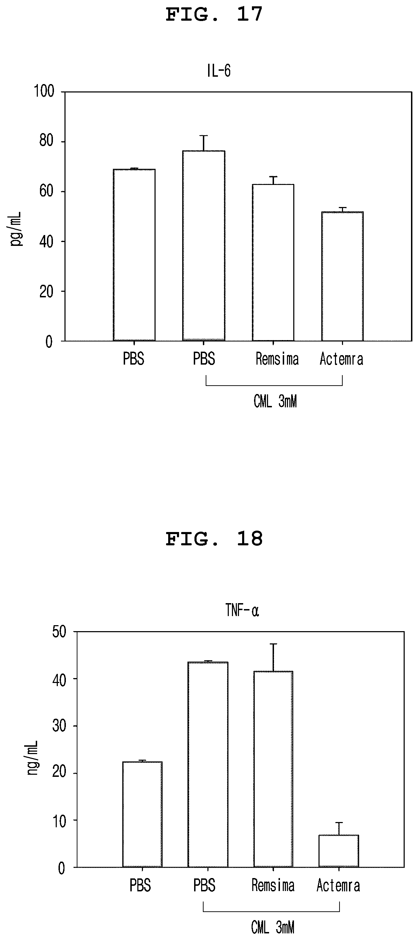

IL-6 was highest in the group treated with CML alone at 76.4±6.1 pg/mL, second highest in the group treated with Remsima (Infliximab) at 62.8±3.1 pg/mL, and lowest in the group treated with Actemra (Tocilizumab) at 51.8±1.8 pg/mL (Figure 17). TNF-α was highest in the group treated with CML alone at 43.5±0.4 ng/mL, second highest in the group treated with Remsima (Infliximab) at 41.6±5.8 ng/mL, and lowest in the group treated with Actemra (Tocilizumab) at 6.8±2.6 ng/mL (Figure 18).

The group treated simultaneously with Actemra (Tocilizumab) showed the lowest SAA, IL-6 and TNF-α concentrations, indicating that the improvement in cytokine storm was most pronounced, which corresponds well with the reduction in inflammatory cell infiltration in the liver tissue. However, the group treated simultaneously with Remsima (Infliximab) showed similar SAA, IL-6 and TNF-α concentrations to the group treated with CML alone. This is interpreted to be related to the minimal effect on improving cytokine storm (Figures 16 to 18).

## Claims

1. A method for screening an anti-inflammatory agent comprising the following steps:
1) a step of inducing hyperinflammation, acute embryotoxicity, and acute cytokine storm by injecting CML into zebrafish embryos 15 to 45 minutes after fertilization;
2) a step of injecting CML together with the test substance into zebrafish embryos 15 to 45 minutes after fertilization;
3) a step of comparing the survival rate of the zebrafish embryos of step 2) with that of the zebrafish embryos of step 1, wherein the developmental stage, the degree of tail elongation, the pigmentation of the eyes, and the presence or absence of the midbrain-hindbrain boundary(MHB) in the brain are confirmed to ensure that the embryos have developed normally; and
4) a step of determining that the zebrafish embryos of step 2) above exhibit normal development if they are at the primordium-3 to primordium-8 stage, have more than 30 somites, have distinct black eye pigmentation, and show a clear midbrain-hindbrain boundary (MHB) in the brain.

2. The method for screening an anti-inflammatory agent according to claim 1, wherein the zebrafish embryos 15 to 45 minutes after fertilization are at the 2-cell stage to 16-cell stage.

3. The method for screening an anti-inflammatory agent according to claim 1, wherein the survival rate of the zebrafish embryos inflamed in step 1) above is 14% to 32%.

4. The method for screening an anti-inflammatory agent according to claim 1, wherein the test substance is selected as an anti-inflammatory agent if the survival rate of the zebrafish embryos of step 2) injected with the test substance is increased than that of the zebrafish embryos of step 1) not injected with the test substance in step 3) above.

5. The method for screening an anti-inflammatory agent according to claim 1, wherein if the zebrafish embryos of step 1) not injected with the test substance remain at the 25-somite stage or less after CML injection, the tail is not elongated, the pigmentation of the eyes is unclear, and the midbrain-hindbrain boundary (MHB) in the brain is not observed, the malformation is considered induced.

6. A method for screening an anti-inflammatory agent comprising the following steps:
1) a step of inducing acute cytokine storm, acute paralysis, and neurotoxicity by injecting CML into zebrafish adults;
2) a step of injecting CML together with the test substance into zebrafish adults;
3) a step of comparing the survival rate of the zebrafish adults of step 2) and that of the zebrafish adults of step 1); and
4) a step of comparing the swimming ability of the zebrafish adults of step 2) and that of the zebrafish adults of step 1).

7. The method for screening an anti-inflammatory agent according to claim 6, wherein the test substance is selected as an anti-inflammatory agent when the infiltration of inflammatory cells in the zebrafish adults of step 2) injected simultaneously with the test substance and the zebrafish adults of step 1) not injected with the test substance is compared by staining cells in the liver tissue, and the infiltration of inflammatory cells in the zebrafish adults of step 2) treated with the test substance is reduced.

8. The method for screening an anti-inflammatory agent according to claim 6, wherein the test substance is selected as an anti-inflammatory agent if the survival rate of the zebrafish adults of step 2), the group injected simultaneously with the test substance, is increased than that of the zebrafish adults of step 1), the untreated control group, in step 3) above.

9. The method for screening an anti-inflammatory agent according to claim 6, wherein the test substance is selected as an anti-inflammatory agent if the swimming ability of the zebrafish adults of step 2), the group injected simultaneously with the test substance, is increased than that of the zebrafish adults of step 1), the untreated control group, in step 4) above.

10. The method for screening an anti-inflammatory agent according to claim 6, wherein the level of total cholesterol, triglyceride, serum amyloid A, IL-6, or TNF-α, which indicates the effect of the test substance in the group injected simultaneously with the test substance of step 2) above, is confirmed to select an anti-inflammatory agent.

11. The method for screening an anti-inflammatory agent according to claim 10, wherein the test substance is selected as an anti-inflammatory agent when the levels of total cholesterol and triglyceride in the test substance co-injected group of step 2) are lower than those in the control group of step 1).

12. The method for screening an anti-inflammatory agent according to claim 10, wherein the test substance is selected as an anti-inflammatory agent when the level of serum amyloid A (SAA), interleukin-6 (IL-6), or tumor necrosis factor-alpha (TNF-α) in the test substance co-injected group of step 2) is lower than that in the control group of step 1).

## Patentansprüche

1. Screening-Verfahren für einen Entzündungshemmer, umfassend die folgenden Schritte:
1) einen Schritt, bei dem Hyperinflammation, akute Embryotoxizität und akute Hyperzytokinämie durch Injektion von CML in Zebrafischembryonen 15 bis 45 Minuten nach der Befruchtung induziert werden;
2) einen Schritt, bei dem CML zusammen mit der Testsubstanz in Zebrafischembryonen 15 bis 45 Minuten nach der Befruchtung injiziert wird;
3) einen Schritt, bei dem die Überlebensrate der Zebrafischembryonen von Schritt 2) mit der der Zebrafischembryonen von Schritt 1) verglichen wird, wobei
das Entwicklungsstadium, der Grad der Schwanzverlängerung, die Pigmentierung der Augen und das Vorhandensein oder Fehlen der Mittelhirn-Hinterhirn-Grenze (MHB) im Gehirn bestätigt werden, um sicherzustellen, dass sich die Embryonen normal entwickelt haben; und
4) einen Schritt, bei dem bestimmt wird, dass die Zebrafischembryonen von Schritt 2) oben normale Entwicklung zeigen, wenn sie sich im Primordium-3- bis Primordium-8-Stadium befinden, mehr als 30 Somiten aufweisen, eine deutliche schwarze Augenpigmentierung aufweisen und eine klare Mittelhirn-Hinterhirn-Grenze (MHB) im Gehirn zeigen.

2. Screening-Verfahren für einen Entzündungshemmer nach Anspruch 1, wobei sich die Zebrafischembryonen 15 bis 45 Minuten nach der Befruchtung im 2-Zellen-Stadium bis 16-Zellen-Stadium befinden.

3. Screening-Verfahren für einen Entzündungshemmer nach Anspruch 1, wobei die Überlebensrate der in Schritt 1) oben entzündeten Zebrafischembryonen bei 14 % bis 32 % liegt.

4. Screening-Verfahren für einen Entzündungshemmer nach Anspruch 1, wobei die Testsubstanz als Entzündungshemmer ausgewählt wird, wenn in Schritt 3) oben die Überlebensrate der mit der Testsubstanz injizierten Zebrafischembryonen von Schritt 2) höher ist als die der nicht mit der Testsubstanz injizierten Zebrafischembryonen von Schritt 1).

5. Screening-Verfahren für einen Entzündungshemmer nach Anspruch 1, wobei, wenn die nicht mit der Testsubstanz injizierten Zebrafischembryonen von Schritt 1) nach CML-Injektion im 25-Somiten-Stadium oder weniger verbleiben, der Schwanz nicht verlängert ist, die Pigmentierung der Augen unklar ist und die Mittelhirn-Hinterhirn-Grenze (MHB) im Gehirn nicht beobachtet wird, die Fehlbildung als induziert betrachtet wird.

6. Screening-Verfahren für einen Entzündungshemmer, umfassend die folgenden Schritte:
1) einen Schritt, bei dem durch Injektion von CML in adulte Zebrafische akute Hyperzytokinämie, akute Lähmung und Neurotoxizität induziert wird;
2) einen Schritt, bei dem CML zusammen mit der Testsubstanz in adulte Zebrafische injiziert wird;
3) einen Schritt, bei dem die Überlebensrate der adulten Zebrafische von Schritt 2) und die der adulten Zebrafische von Schritt 1) verglichen werden; und
4) einen Schritt, bei dem die Schwimmfähigkeit der adulten Zebrafische von Schritt 2) und die der adulten Zebrafische von Schritt 1) verglichen werden.

7. Screening-Verfahren für einen Entzündungshemmer nach Anspruch 6, wobei die Testsubstanz als Entzündungshemmer ausgewählt wird, wenn die Infiltration von Entzündungszellen in den adulten Zebrafischen von Schritt 2), die gleichzeitig mit der Testsubstanz injiziert wurden, und den adulten Zebrafischen von Schritt 1), die nicht mit der Testsubstanz injiziert wurden, durch Färben von Zellen im Lebergewebe verglichen wird und die Infiltration von Entzündungszellen bei den mit der Testsubstanz behandelten adulten Zebrafischen von Schritt 2) verringert ist.

8. Screening-Verfahren für einen Entzündungshemmer nach Anspruch 6, wobei die Testsubstanz als Entzündungshemmer ausgewählt wird, wenn in Schritt 3) oben die Überlebensrate der adulten Zebrafische von Schritt 2), der Gruppe, die gleichzeitig mit der Testsubstanz injiziert wurde, höher ist als die der adulten Zebrafische von Schritt 1), der unbehandelten Kontrollgruppe.

9. Screening-Verfahren für einen Entzündungshemmer nach Anspruch 6, wobei die Testsubstanz als Entzündungshemmer ausgewählt wird, wenn in Schritt 4) oben die Schwimmfähigkeit der adulten Zebrafische von Schritt 2), der Gruppe, die gleichzeitig mit der Testsubstanz injiziert wurde, größer ist als die der adulten Zebrafische von Schritt 1), der unbehandelten Kontrollgruppe.

10. Screening-Verfahren für einen Entzündungshemmer nach Anspruch 6, wobei zum Auswählen eines Entzündungshemmers der Spiegel von Gesamtcholesterin, Triglycerid, Serumamyloid A, IL-6 oder TNF-α, der die Wirkung der Testsubstanz in der gleichzeitig mit der Testsubstanz injizierten Gruppe von Schritt 2) oben anzeigt, bestätigt wird.

11. Screening-Verfahren für einen Entzündungshemmer nach Anspruch 10, wobei die Testsubstanz als Entzündungshemmer ausgewählt wird, wenn die Spiegel von Gesamtcholesterin und Triglycerid bei der zusammen mit Testsubstanz injizierten Gruppe von Schritt 2) niedriger sind als die bei der Kontrollgruppe von Schritt 1).

12. Screening-Verfahren für einen Entzündungshemmer nach Anspruch 10, wobei die Testsubstanz als Entzündungshemmer ausgewählt wird, wenn der Spiegel von Serumamyloid A (SAA), Interleukin-6 (IL-6) oder Tumornekrosefaktor-alpha (TNF-α) bei der zusammen mit Testsubstanz injizierten Gruppe von Schritt 2) niedriger ist als der bei der Kontrollgruppe von Schritt 1).

## Revendications

1. Procédé de criblage d'un agent anti-inflammatoire comprenant les étapes suivantes :
1) une étape d'induction d'une hyperinflammation, d'une embryotoxicité aiguë et d'un orage cytokinique aigu par injection de CML dans des embryons de poisson zèbre 15 à 45 minutes après la fécondation ;
2) une étape d'injection de CML avec la substance d'essai dans des embryons de poisson zèbre 15 à 45 minutes après la fécondation ;
3) une étape de comparaison du taux de survie des embryons de poisson zèbre de l'étape 2) à celui des embryons de poisson zèbre de l'étape 1, dans lequel
le stade de développement, le degré d'allongement de la queue, la pigmentation des yeux, et la présence ou l'absence de la jonction mésencéphale-rhombencéphale (MHB) dans le cerveau sont confirmés afin de s'assurer du développement normal des embryons ; et
4) une étape de détermination du fait que les embryons de poisson zèbre de l'étape 2) ci-dessus présentent un développement normal s'ils sont au stade primordium-3 à primordium-8, ont plus de 30 somites, ont une pigmentation noire distincte des yeux, et présentent une jonction mésencéphale-rhombencéphale (MHB) nette dans le cerveau.

2. Procédé de criblage d'un agent anti-inflammatoire selon la revendication 1, dans lequel les embryons de poisson zèbre, 15 à 45 minutes après la fécondation, sont au stade de 2 cellules à 16 cellules.

3. Procédé de criblage d'un agent anti-inflammatoire selon la revendication 1, dans lequel le taux de survie des embryons de poisson zèbre présentant une inflammation dans l'étape 1) ci-dessus est de 14 % à 32 %.

4. Procédé de criblage d'un agent anti-inflammatoire selon la revendication 1, dans lequel la substance d'essai est sélectionnée comme agent anti-inflammatoire si le taux de survie des embryons de poisson zèbre de l'étape 2) ayant reçu une injection de la substance d'essai est augmenté par rapport à celui des embryons de poisson zèbre de l'étape 1) n'ayant pas reçu d'injection de la substance d'essai de l'étape 3) ci-dessus.

5. Procédé de criblage d'un agent anti-inflammatoire selon la revendication 1, dans lequel, si les embryons de poisson zèbre de l'étape 1) n'ayant pas reçu d'injection de la substance d'essai restent au stade 25 somites ou moins après injection de CML, la queue n'est pas allongée, la pigmentation des yeux n'est pas nette et la jonction mésencéphale-rhombencéphale (MHB) dans le cerveau n'est pas observée, la malformation est considérée comme induite.

6. Procédé de criblage d'un agent anti-inflammatoire comprenant les étapes suivantes :
1) une étape d'induction d'un orage cytokinique aigu, d'une paralysie aiguë et d'une neurotoxicité par injection de CML chez des poissons zèbres adultes ;
2) une étape d'injection de CML avec la substance d'essai à des poissons zèbres adultes ;
3) une étape de comparaison du taux de survie des poissons zèbres adultes de l'étape 2) à celui des poissons zèbres adultes de l'étape 1) ; et
4) une étape de comparaison de la capacité de nage des poissons zèbres adultes de l'étape 2) à celle des poissons zèbres adultes de l'étape 1).

7. Procédé de criblage d'un agent anti-inflammatoire selon la revendication 6, dans lequel la substance d'essai est sélectionnée comme agent anti-inflammatoire lorsque l'infiltration de cellules inflammatoires chez les poissons zèbres adultes de l'étape 2) ayant reçu simultanément une injection de la substance d'essai et les poissons zèbres adultes de l'étape 1) n'ayant pas reçu une injection de la substance d'essai est comparée par coloration de cellules dans le tissu hépatique. et l'infiltration de cellules inflammatoires chez les poissons zèbres adultes de l'étape 2) traités avec la substance d'essai est réduite.

8. Procédé de criblage d'un agent anti-inflammatoire selon la revendication 6, dans lequel la substance d'essai est sélectionnée comme agent anti-inflammatoire si le taux de survie des poissons-zèbres adultes de l'étape 2), le groupe ayant reçu simultanément une injection de la substance d'essai, est augmenté par rapport à celui des poissons-zèbres adultes de l'étape 1), le groupe témoin non traité, dans l'étape 3) ci-dessus.

9. Procédé de criblage d'un agent anti-inflammatoire selon la revendication 6, dans lequel la substance d'essai est sélectionnée comme agent anti-inflammatoire si la capacité de nage des poissons-zèbres adultes de l'étape 2), le groupe ayant reçu simultanément une injection de la substance d'essai, est augmentée par rapport à celui des poissons-zèbres adultes de l'étape 1), le groupe témoin non traité, dans l'étape 4) ci-dessus.

10. Procédé de criblage d'un agent anti-inflammatoire selon la revendication 6, dans laquelle le taux de cholestérol total, de triglycérides, d'amyloïde A sérique, d'IL-6, ou de TNF-α, qui indique l'effet de la substance d'essai dans le groupe ayant reçu simultanément une injection de la substance d'essai de l'étape 2) ci-dessus, est confirmé pour sélectionner un agent anti-inflammatoire.

11. Procédé de criblage d'un agent anti-inflammatoire selon la revendication 10, dans lequel la substance d'essai est sélectionnée comme agent anti-inflammatoire lorsque les taux de cholestérol total et de triglycérides dans le groupe ayant reçu une co-injection de substance d'essai de l'étape 2) sont inférieurs à ceux du groupe témoin de l'étape 1).

12. Procédé de criblage d'un agent anti-inflammatoire selon la revendication 10, dans lequel la substance d'essai est sélectionnée comme agent anti-inflammatoire lorsque le taux d'amyloïde A sérique (SAA), d'interleukine-6 (IL-6) ou de facteur de nécrose tumorale alpha (TNF-α) dans le groupe ayant reçu une co-injection de substance d'essai de l'étape 2) est inférieur à celui du groupe témoin de l'étape 1).
